# EUROPEAN PATENT SPECIFICATION

(11) **EP 1 029 074 B1**
(45) Date of publication and mention of the grant of the patent: **01.12.2004**
(21) Application number: 99936969.7
(22) Date of filing: 27.07.1999
(51) Int. Cl.: C12Q 1/34, C12Q 1/37, C12Q 1/44, C12N 15/12, C12Q 1/04, C12Q 1/06, C12Q 1/10, C12Q 1/14

(54) **DIFFERENTIAL STAINING OF BACTERIA USING FLUORESCENCE**
DIFFERENZIERUNGSFÄRBUNG VON BAKTERIEN UNTER VERWENDUNG VON FLUORESZENZ
COLORATION DIFFERENTIELLE DE BACTERIES PAR FLUORESCENCE

(30) Priority: 29.07.1998 IT PE980017
(43) Date of publication of application: 23.08.2000
(73) Proprietor: Fazii, Paolo, 65100 Pescara (IT); Ciancaglini, Ettore, 65100 Pescara (IT)
(72) Inventor: Fazii, Paolo, 65100 Pescara (IT); Ciancaglini, Ettore, 65100 Pescara (IT)
(74) Representative: Di Blasio, Gianluca
(86) International application number: PCT/IT1999/000238
(87) International publication number: WO 2000/006765

(56) References cited:
- US-A- 4 665 024

## Description

### BACKGROUND OF THE INVENTION

### Field of the invention

The present application relates to the staining of microorganisms. More particularly, it relates to a differential staining method which distinguishes gram-positive from gram-negative bacteria in fluorescence.

### Description of the prior art

The Gram stain was originally developed to distinguish bacteria that are more resistant to decolourization (gram-positive bacteria) from bacteria that are less resistant to decolourization (gram-negative bacteria). This staining method permits the differentiation of bacteria into two groups, based on the colour of the stained bacteria at the conclusion of the staining procedure.

The Gram stain is performed on bacterial specimens which are applied to glass slides, air dried and fixed with heat

Staining of bacteria with fluorescent dyes is well known. McCarthy and Senne (Evaluation of acridine orange stain for detection of microorganisms in blood cultures. Journal of Clinical Microbiology, 1980, 11:281-285) reported of a fluorescent staining method to detect microorganisms in direct smears prepared from clinical specimens (e.g. blood). In this report, the acridine orange stain resulted more sentitive than Gram stain for the detection of bacteria in blood.

For the safety of the operators, in world-wide medical practice the staining methods are usually performed on samples containing pathogenous bacteria which are previously applied to the slides, air dried and fixed with heat, rapidly passing the slides through a flame.

U.S. Pat. No. 4665024 to Mansour concerned a staining method for the determination of Gram sign in suspensions of live pathogenous bacteria, using ethidium bromide - acridine orange and ethidium bromide - thioflavin T or ethidium bromide and rhodamine 123, 3,3-dipentyloxacarbocyanine iodide and rhodamine B, without any decolorization. The analysis of the suspensions was carried out by spectrofluorometry or by flow microfluorometry techniques. In an alternative embodiment of the invention, the living microorganisms were stained with a single fluorescent dye. To identify the unstained live gram-negative bacteria was used a light microscope. A fluorescence microscope was used to identify the stained live gram-positive bacteria.

### DETAILED DESCRIPTION OF THE INVENTION

The present application is directed to differential staining of gram-positive and gram-negative bacteria using fluorescent stains.

The invention appears to comprise the following essential technical features:
staining a sample containing bacteria with acridine orange;
treating the stained sample with a mixture of alcohol/acetone;
staining the sample from step (b) with fluorescein;
discriminating between gram-positive and gram-negative bacteria by means of fluorescent microscopy.

This method can be applied to clinical specimens and is based on two fluorochromes, one acting in the wavelength of red, and another acting in the wavelength of green, which together form a red/green system. In previous experiments we noted that gram-positive and gram-negative bacteria stained with acridine orange are decolourized differently by ethyl alcohol/acetone. To intensify these differences we used a red/green system (red + green = yellow) by adding a second green fluorochrome, fluorescein. Bacteria that are more resistant to decolourization (gram-positive bacteria) will retain a sufficient amount of acridine orange and will appear yellow when the red fluorochrome is added to the second (green) fluorochrome. In contrast, bacteria that are less resistant to decolourization (gram-negative bacteria) will not retain enough acridine orange and will appear green.

The slides were prepared by first placing a drop of distilled water and then applying the sample containing bacteria. The smears were subsequently air dried and fixed with heat.

Staining Procedure.

First, the slide was covered with the acridine orange solution for 2 min to allow the stain to act.

Afterward, the slide was rinsed in tap water. The ethyl alcohol/acetone solution was applied and was allowed to act for exactly 10 s. Next, the slide was rinsed again in tap water. Then, the slide was covered with the fluorescein solution for 2 min to allow the stain to act. After a final rinse in tap water, the slide was air dried and examined using a fluorescence microscope.

Using the differential staining method in fluorescence, the gram-positive bacteria appear yellow and the gram-negative bacteria appear green.

The following example is provided to further illustrate the invention, but is not to be construed in any way as limitative of the invention.

Microorganisms.

The slides were prepared by first placing a drop of distilled water and then applying the samples of culture. The smears were subsequently air dried and fixed with heat. The bacterial species were obtained from routine cultures and were grown for 24-48 h. The following 25 strains of gram-positive and gram-negative bacterial species were tested.

| **GRAM-POSITIVE BACTERIA** | |
|---|---|
| Staphylococcus aureus | (5 strains) |
| Streptococcus pyogenes | (2 strains) |
| Enterococcus sp. | (4 strains) |
| Corynebacterium sp. | (1 strain) |

| **GRAM-NEGATIVE BACTERIA** | |
|---|---|
| Escherichia coli | (4 strains) |
| Klebsiella pneumoniae | (1 strain) |
| Klebsiella sp. | (3 strains) |
| Enterobacter sp. | (4 strains) |
| Pseudomonas sp. | (1 strain) |

At the conclusion of the staining procedure all the gram-positive bacteria appeared yellow and all the gram-negative bacteria appeared green.

The present disclosure is to be considered as exemplary of the principles of the invention, and is not limited to the embodiments illustrated. Any microorganism can be determined by the method of the invention as, for example, gram-positive bacteria such as Staphylococcus aureus, Streptococcus pyogenes, Enterococcus sp. , and the like, and gram-negative bacteria such as Escherichia coli, Klebsiella pneumoniae, Pseudomonas sp., and the like. The microorganisms to be stained may be from any source. For example they may be present in a body fluid. This staining method can be used as screening method to detect bacteria in smears prepared from urine and can be used for the detection of bacteria in blood and in direct smears of cerebrospinal fluid.

In order to ensure reproducible staining, several guidelines must be followed. The acridine orange staining reagent must be prepared by adding the fluorescent dye to the acetate buffer in sufficient quantity to provide a final dye concentration in the staining solution of from about 0.01% to about 0.7%, preferably about 0.7%. Ethyl alcohol/acetone was prepared as a 50%-50% solution. The sodium fluorescein must be dissolved in a suitable solvent, preferably ethyl alcohol, in sufficient quantity to provide a final dye concentration of 0.002%. A staining time of 2 min was found to be optimal for both dyes. The acridine orange solution must be buffered at low pH, preferably pH 4, because a slight variation in colour and intensity occurs at every 0.5 pH value difference. Also the sodium fluorescein solution must be buffered at low pH, preferably pH 5.5-6. The fluorescein solubility is modified by pH value. The following aspects of the procedure must be accurate: staining times, decolourization times and wash times; above all, the decolourization time must be accurate. Finally, the staining method should be performed on young (24-48 h) cultures.

The use of the combination acridine orange, ethyl alcohol/acetone and fluorescein to differentiate between gram-positive and gram-negative bacteria is neither disclosed nor suggested in the cited prior art. The method which is described in U.S. Pat. No. 4665024 teaches only the use of ethidium bromide in combination with further agents including acridine orange.

The present application differs from the method which is described in U.S. Pat. No. 4665024 because:
A) The fluorescent dyes are different. We have used the acridine orange in combination with fluorescein to form a chromatic system, the red/green system (e.g. red+green=yellow). Instead the U.S. Pat. No. 4665024 described a method which did not use the fluorescein but used other dyes. Furthermore the method which is described in U.S. Pat. No. 4665024 used the acridine orange in a mixture with ethidium bromide and not in combination with fluorescein. The ethidium bromide and the acridine orange do not together form a red/green system.
B) The colours obtained are different. Using our method the gram-positive bacteria appear yellow and the gram-negative bacteria appear green. Instead, the U.S. Pat. No. 4665024 described a method which obtained gram-positive bacteria "stained substantially orange" and gram-negative bacteria "stained substantially green" but also gram-negative bacteria unstained or gram-positive bacteria "more red than green" and gram-negative bacteria "more green than red", in the last case was not used a fluorescence microscope and the analysis was carried out by spectrofluorometry or by flow microfluorometry techniques.
C) The staining procedure is different. Our method is based on the following procedure. First, the acridine orange solution was used to stain gram-positive and gram-negative bacteria. Afterward the slide was rinsed in tap water. The decolourizer was used to differentiate between gram-positive and gram-negative bacteria. Next, the slide was rinsed again in tap water. Then, the fluorescein solution was used to intensify the differences between gram-positive and gram-negative bacteria. After a final rinse in tap water, the slides were air dried and examined using a fluorescence microscope. As in Gram stain, the decolorization is essential to enable differentiation between gram-positive and gram-negative bacteria. Instead, the U.S. Pat. No. 4665024 described a method which used one or more fluorescent dyes which are added simultaneously or sequentially and did not use any decolourization.
D) The observed objects are biologically different We stained bacterial specimens which were previously applied to the slides air dried and fixed with heat, rapidly passing the slides through a flame. The method which is described in U.S. Pat. No. 4665024 used living bacteria in suspension or in dry smear, but never used bacteria which were fixed with heat or with other method.
E) A chromatic system producing a overstain differs from a mixture. In a chromatic system, two colours are added in known percentage and together form a third different colour. We used a chromatic system, the red/green system (e.g. red+green=yellow) to intensify the differences produced on bacteria by decolourization and to obtain a overstain. Instead the method which is described in U.S. Pat. No. 4665024 did not use any decolourization and any chromatic system to produce a overstain but used a mixture of fluorescent dyes. The mixture of certain fluorescent dyes does not form any chromatic system.
F) The instruments are different. Our method is a fluorescent staining method for optical fluorescence microscope. Instead the U.S. Pat. No. 4665024 described a method which used optical and non-optical instruments: spectrofluommetry technique, flow microfluorometry technique, light microscope and fluorescence microscope.
G) In sum, the only point of contact of the two methods is the use of fluorescent dyes.

This differential staining method in fluorescence is simple to use, has no toxicity for the operators, moreover the differences between gram-positive and gram-negative bacteria are always clear and easy to detect This method provides significant advantages over the conventional Gram stain because it is more sensitive than the Gram stain and can be used in clinical microbiology, laboratory routine and microbiological research. The microorganisms appeared to stain well by this method. A marked differential staining effect and an excellent contrast between stained bacteria and the background were obtained. The microorganisms were easily detected even if only small numbers of bacteria were present The differential fluorescent staining method can be used as screening method to detect microorganisms in smears prepared from urine and can also be used on direct smears of cerebrospinal fluid. In view of the importance of a rapid aetiological diagnosis in cases of septicaemia, the differential staining method in fluorescence may also be used for the detection of bacteria in blood.

## Claims

1. Method for discriminating between Gram-positive and Gram-negative bacteria, comprising:
a) staining a sample containing bacteria with acridine orange;
b) treating the stained sample with a mixture of alcohol/acetone;
c) staining the sample from step b) with fluorescein;
d) discriminating between Gram-positive and Gram-negative bacteria by means of fluorescent microscopy.

## Patentansprüche

1. Eine Methode für die Differenzierung von grampositiv von gramnegativ Keimen bestehend aus:
a) Färbung eines Ausstrichs mit Keimen mit Acridine Orange
b) Der gefärbte Ausstrich wird mit Alkohol/Aceton behandelt
c) Färbung des Ausstrich nach dem Schritt b) mit Fluoreszein
d) Differenzierung der grampositiven von gramnegativen Keimen durch If- Mikroskop

## Revendications

1. Un méthode pour différencier les germes Gram positifs des germes Gram négatifs qui comprendre :
a) la coloration de l'étalement qui contenant les germes avec l'acridine orange
b) l'étalement coloré est traité avec un complexe alcool/acétone
c) la coloration de l'étalement avec la fluorescéine après le passage b)
d) les germes Gram positifs et les germes Gram négatifs se différencient avec un microscope á fluorescence.
